# EUROPEAN PATENT SPECIFICATION

(11) **EP 1 913 391 B1**
(45) Date of publication and mention of the grant of the patent: **29.12.2010**
(21) Application number: 06801170.9
(22) Date of filing: 10.08.2006
(51) Int. Cl.: G01N 33/53

(54) **QUANTITATIVE ASSAYS FOR PDGFR-beta IN BODY FLUIDS**
QUANTITATIVE ASSAYS FÜR PDGFR-beta- IN KÖRPERFLÜSSIGKEITEN
DOSAGES QUANTITATIFS DE PDGFR-beta DANS DES FLUIDES CORPORELS

(30) Priority: 11.08.2005 US 707806 P
(43) Date of publication of application: 23.04.2008
(73) Proprietor: Siemens Healthcare Diagnostics Inc., Tarrytown, NY 10591 (US)
(72) Inventor: HAMER, Peter, J., Reading, MA 01867 (US); CARNEY, Walter, P., North Andover, MA 01845 (US); MORRIS, Leticia, Chestnut Hill, MA 02467 (US); ELTING, James, Madison, CT 06643 (US)
(74) Representative: Maier, Daniel Oliver
(86) International application number: PCT/US2006/031245
(87) International publication number: WO 2007/021860

(56) References cited:
- EP-A- 0 869 177
- WO-A-2004/113274
- WO-A-2005/015236
- US-A1- 2003 219 842
- US-A1- 2003 219 842
- US-B1- 6 264 949
- FAMULOK M.: 'Bringing Picomolar Protein Detection into Proximity' NATURE BIOTECHNOLOGY vol. 20, May 2002, pages 448 - 449, XP002375041

## Description

### FIELD OF THE INVENTION

The present invention is in the general area of medical genetics and in the fields of oncology and immunology. More specifically, the invention is directed to the detection and quantification of total PDGFR-β in body fluids, particularly serial changes of total PDGFR-β levels in a subject's body fluids. Further, the invention is directed to detecting and quantitating total PDGFR-β in conjunction with one or more other proteins, such as, oncoproteins, angiogenic factors, tumor markers, inhibitors, growth factor receptors, metastasis proteins, and tumor suppressors.

### BACKGROUND

Platelet-derived growth factor receptors (PDGFRs) and their ligands, platelet-derived growth factors (PDGFs), play critical roles in mesenchymal cell migration and proliferation. The PDGFR/PDGF system includes two receptors (PDGFR-α and PDGFR-β) and four ligands (PDGF-A, B, C and D) [Hart et al. 1988]. The receptors are plasma membrane-spanning proteins with intracellular tyrosine kinase domains. As with other protein kinases, activation of the PDGFRs is a key mechanism in regulating signals for cell proliferation, and abnormalities of PDGFR/PDGF are thought to contribute to a number of human diseases, such as atherosclerosis, balloon injury induced restenosis, pulmonary fibrosis, liver fibrosis, and especially malignancy.

The two receptors PDGFR-α and PDGFR-β are related in sequence (30% amino acid similarity), and have a common overall structure having four domains: an extracellular ligand binding domain consisting of five immunoglobulin-like structures, a transmembrane domain, a regulatory juxtamembrane domain and an intracellular catalytic domain. Both receptors are members of the class III subtype of receptor tyrosine kinases (RTKs), a group that shares a characteristic insertion sequence between two conserved elements of the tyrosine kinase domain. Normally, the receptors require dimerization (induced by binding of PDGF dimers) for autophosphorylation and activation. The PDGFR-β receptor strongly binds only the BB and DD dimers of PDGF, whereas the PDGFR-α receptor binds AA, BB and CC homodimers and AB heterodimers with similar affinity, but binds only weakly to DD homodimers [Jones and Cross 2004].

In embryogenesis the PDGFR/PDGF system is essential for the correct development of the kidney, cardiovascular system, brain, lung and connective tissue. In adults, PDGFR/PDGF is important in wound healing, inflammation and angiogenesis. Human dermal fibroblasts appear to express seven times as much PDGFR-β receptor as PDGFR-α/β receptor, and the PDGFR-β receptor is responsible for most PDGF receptor phosphorylation [Hart et al. 1988].

Many diseases have been associated with dysregulated PDGFR function: for example, constitutive activation of the PDGFR-α or PDGFR-β receptor tyrosine kinases is seen in myeloid malignancies as a consequence of fusion to diverse partner genes, and activating mutations of PDGFR-α are seen in gastrointestinal stromal tumours (GISTs). The loss of von Hippel-Lindau disease protein (pVHL) in clear-cell renal carcinoma (RCC) leads to dysregulation of the PDGF-P/PDGFR-β pathway, among other pathways. [Gollob 2005]. Overexpression of PDGFRs and/or their ligands has been described in many solid tumours, including some brain tumors [George 2001; Fleming et al. 1992; Smith et al. 2000; MacDonald et al. 2001 (PDGFR-β erroneously first identified as PDGFR-α)]. Recently, two independent laboratories have reported that PFGFR-β expression is an indication of activated hepatic stellate cells (HPC), a marker of liver fibrosis [Breitkopf et al., 2005; Czochra et al., 2006]. In some cases, like chronic monomyelocytic leukemia (CMML), the persistent PDGFR signaling is essential for the survival of the cancer cell [Levitski 2004].

It is unclear whether overexpression of PDGFRs is really a primary cause of these diseases, but overexpression of receptor tyrosine kinases are observed in a wide variety of human cancers, and is believed to result in receptor activation by crowding at the plasma membrane and consequent ligand-independent dimerization [Jones and Cross 2004]. Agents that block PDGFR signaling have recently been found, such as ST1571 [also known as imatinib mesylate or Gleevec®]. That compound has clear clinical activity in patients with myeloid malignancies, gastrointestinal tumors (GISTs) and dermatofibrosarcoma protruberans (DFSP) [Jones and Cross 2004]. Furthermore, it has been shown that inhibition of PDGFRs by imatinib may enhance the uptake of chemotherapeutic drugs by lowering interstitial hypertension [Pietras et al. 2002], and therefore imatinib may be used as a general strategy to enhance the effects of chemotherapy [Jones and Cross 2004].

It would be useful to have a noninvasive assay for total circulating PDGFR-β levels in humans (i.e., both tyrosine-phosphorylated and unphosphorylated), as the detection of changes in such levels is a potentially important diagnostic, prognostic, and therapeutic tool. In an animal model, one group has described the detection of a rat PDGFR-β/IgG fusion protein at about 100 ng/ml in rat sera of adenoviral vector-transfected rats, using commercially-available antibodies in an ELISA [Borkham-Kamphorst et al. 2004]. Although immunohistochemical assays of both PDGF receptors and quantitative assays of circulating PDGFR-α have been used in humans (Tiesman and Hart 1993), until the instant disclosure, no quantitative assays for circulating PDGFR-β have been reported.

### SUMMARY OF THE INVENTION

The invention concerns the methods of claims 1, 3 and 7. Serial changes are particularly useful as an indicator of the activation status of the PDGF/PDGFR signal transduction pathway, wherein "activation" is defined to include both upregulation and mutational activation of PDGFR-β. According to the theories underlying the methods of the invention, increasing total PDGFR-β protein (detected by serial changes in total PDGFR-β levels) is considered to be the predominant indicator of an activated PDGF/PDGFR signal transduction pathway, signifying the presence of disease In a human subject. In some instances, decreasing total PDGFR-β protein may be an indicator of a dysregulated PDGF/PDGFR signal transduction pathway.

The methods of the invention can be used clinically - diagnostically and/or prognostically, to detect precancer and/or cancer (preneoplastic/neoplastic disease), as a therapeutic aid for patient therapy selection, to monitor the status of a preneoplastic/neoplastic disease in a patient, and/or to monitor how a patient with a preneoplastic/neoplastic disease is responding to a therapy. A preferred use for the immunoassays of the invention is to monitor the status of patients and how patients are responding to therapy, and to make decisions about the optimal method for patient therapy.

The sample is serum or plasma.

In one aspect, the invention concerns the use of an immunoassay as a method of monitoring the status of a disease in a patient, which is a preneoplastic/neoplastic disease, and/or monitoring how a patient with a disease is responding to a therapy, comprising immunologically detecting and quantifying serial changes in total PDGFR-β protein levels in samples of a body fluid taken from said patient over time; wherein increasing levels of total PDGFR-β protein over time indicate disease progression or a negative response to said therapy, and wherein decreasing levels of total PDGFR-β protein indicate disease remission or a positive response to said therapy. Said therapy may be conventional anticancer therapy, unconventional anticancer therapy, and/or a PDGF pathway-directed therapy. A preferred body fluid sample, among other types of samples, may be a pretreatment sample, or a sample from a cancer patient who has not responded to treatment.

Said method of monitoring the status of a disease associated with an activated PDGF pathway in a human subject may be further prognostic for said disease, wherein the levels of total PDGFR-β protein in the subject's samples relative to the average level of total PDGFR-β in the control samples are indicative of a better or poorer prognosis for said subject. Preferably, said prognosis is a clinical outcome selected from the group consisting of response rate (RR), complete response (CR), partial response (PR), stable disease (SD), clinical benefit [including complete response (CR), partial response (PR), and stable disease (SD)], time to progression (TTP), progression free survival (PFS) and overall survival (OS).

Said methods can be in standard formats by an immunoassay in the form of a sandwich immunoassay, preferably a sandwich enzyme-linked immunosorbent assay (ELISA). A preferred format is a sandwich ELISA comprising the use of monoclonal antibodies, preferably anti-total PDGFR-β monoclonal antibodies, preferably monoclonal antibodies that specifically bind the ectodomain of PDGFR-β Another preferred format is a sandwich ELISA comprising the use of a monoclonal antibody as the capture antibody and a polyclonal antibody as the detector antibody. In either of those preferred formats, preferably, the detector antibody, whether polyclonal or monoclonal, is biotinylated. A preferred capture antibody Is the mouse anti-human PDGFR-β monoclonal antibody, AC5.1.3, which was prepared against recombinant wild-type total PDGFR-β using standard Kohler-Milstein technologies.

An exemplary and preferred ELISA sandwich immunoassay comprises the use of a mouse anti-human PDGFR-β monoclonal antibody as the capture antibody and a goat or rabbit anti-human PDGFR-β polyclonal antibody as the detector antibody. In such an exemplary embodiment, the detection means may comprise the use of an enzyme labeled secondary antibody, preferably a horseradish peroxidase-labeled secondary antibody, which binds to the detector antibody. Alternatively, the detector polyclonal antibody may be conjugated with biotin prior to binding with a strepavidin-enzyme, preferably strepavidin-horseradish peroxidase, or may be directly labeled with an enzyme, such as with horseradish peroxidase (HRP).

Exemplary of enzyme-substrate combinations that may be used to label the detector antibody are horseradish peroxidase and tetramethyl benzidine (TMB), and alkaline phosphatase and paranitrophenyl phosphate (pNPP). Preferably, the enzyme used in the sandwich ELISA is horseradish peroxidase (HRP), its substrate being TMB.

Said methods can be diagnostic and/or prognostic for cancerous or precancerous diseases, wherein said disease is associated with an activated PDGF pathway. Exemplary precancerous or cancerous diseases associated with an activated PDGF pathway are those selected from the group consisting of metastatic medulloblastoma, gastrointestinal stromal tumors (GISTs), dermatofibrosarcoma protruberans (DFSP), colorectal cancer, colon cancer, lung cancer, non-small-cell lung cancer, small-cell lung cancer, acute myelogenous leukemia, thyroid cancer, pancreatic cancer, bladder cancer, kidney cancer, melanoma, breast cancer, prostate cancer, ovarian cancer, cervical cancer, head-and-neck cancer, brain tumors, hepatocellular carcinoma and hematologic malignancies, and precancers leading to such cancers. Colon, colorectal, lung cancer, prostate cancer and breast cancer, and precancers leading thereto, are considered particular targets for the diagnostic/prognostic assay methods that detect serial changes in total PDGFR-β in human body fluids.

Chronic myeloproliferative diseases (CMPDs) are also exemplary of diseases associated with an activated PDGF pathway. Chronic myeloproliforative diseases may be classified as non-neoplastic or as preneoplastic/neoplastic, but are usually considered as preneoplastic/neoplastic, more usually as neoplastic.

A second aspect of the invention concerns a quantitative immunoassay to measure serial changes in the levels of total PDGFR-β protein in human body fluids, as a method of therapy selection for a human patient with a preneoplastic/neoplastic disease. One such method of therapy selection comprises the steps of:
(a) immunologically detecting and quantifying the average level of total PDGFR-β protein in samples of a body fluid taken from individuals of a control population;
(b) immunologically detecting and quantifying serial changes in total PDGFR-β protein levels in equivalent samples of body fluid taken from the patient over time;
(c) comparing the levels of total PDGFR-β protein in the patient's samples to the average level of total PDGFR-β protein in the control samples; and
(d) deciding whether to use conventional therapy and/or PDGF pathway-directed therapy to treat the patient based upon the differences between the levels of total PDGFR-β protein in the patient's samples and the average level of total PDGFR-β protein in the control samples, and in view of the serial changes among the levels of total PDGFR-β protein in the patient's samples. For example, if a level of total PDGFR-β protein in a patient's sample is found to be above the average level of total PDGFR-β protein in the control samples, and if the serial changes in the total PDGFR-β protein levels in the patient's samples reflect levels that are trending above the average level of total PDGFR-β protein in the control samples, the conclusion could be drawn that the patient has a PDGF driven disease, and the decision may be made to use PDGF pathway-directed therapy to treat the patient, either alone or in conjunction with one or more other therapies.

Preferably, said PDGF pathway-directed therapy is selected from the group consisting of multi-kinase inhibitors, tyrosine kinase inhibitors, bis-aryl ureas, antisense inhibitors of PDGFR, and monoclonal antibody therapies. More preferably, said PDGF pathway-directed therapy is the bis-aryl urea Sorafenib (BAY 43-9006), which is an angiogenesis inhibitor as well as a tyrosine kinase inhibitor, or the tyrosine kinase inhibitor STI571 [also known as imatinib mesylate or Gleevec®.] An exemplary monoclonal antibody therapy is CDP860, which is a humanized di-Fab' fragment against PDGFR-β.

Another aspect of this invention concerns diagnostic/prognostic methods to detect a preneoplastic/neoplastic disease, associated with an activated PDGF pathway. One method of detecting a disease associated with an activated PDGF pathway in a human subject comprises the steps of:
(a) immunologically detecting and quantifying the average level of total PDGFR-β protein in samples of a body fluid taken from individuals of a control population;
(b) immunologically detecting and quantifying serial changes in total PDGFR-β protein levels in equivalent samples of body fluid taken from the subject over time; and
(c) comparing the levels of total PDGFR-β protein in the subject's samples to the average level of total PDGFR-β protein in the control samples;
wherein a level of total PDGFR-β protein in the subject's samples that is above the average level of total PDGFR-β protein in the control samples Is indicative of an activated PDGF pathway and the presence of disease in the subject.

Still another aspect of the invention concerns the use of quantitative immunoassays to detect changes in total PDGFR-β levels in combination with the levels of one or more other protein(s), in the methods of this invention as in the diagnostic/prognostic methods, in the methods of therapy selection for patients with a disease, in the methods of monitoring the status of a disease in a patient, and of monitoring how a patient with a disease is responding to a PDGF pathway-directed or other therapy, wherein said disease is cancer or precancer. Preferably, said other proteins that would be useful to quantitate along with PDGFR-β in the assays of this invention are inhibitors, oncoproteins, growth factor receptors, angiogenic factors, metastasis proteins, tumor markers and tumor suppressors. Preferably, said inhibitor is tissue-inhibitor of metalloproteinase-1 (TIMP-1), said oncoproteins are selected from the group consisting of HER-2/neu and ras p21, said growth factor receptors are selected from the group consisting of epidermal growth factor receptor (EGFR) and platelet derived growth factor receptor alpha (PDGFR-α), said angiogenic factor is vascular endothelial growth factor (VEGF), said metastasis protein is urokinase-type plasminogen activator (uPA), said tumor marker is carcinoembryonic antigen (CEA), and said tumor suppressor is p53. It would be advantageous to test patients, preferably cancer patients, for serial changes in both total PDGFR-β and such other proteins, especially proteins that activate the PDGF pathway, to enlarge the clinical perspective, therapeutic resources and diagnostic/prognostic parameters to pick the optimal therapeutic combinations for the most promising treatment outcomes.

### REFERENCES

- Breitkopf et al., Cytokine, 31 (5): 349-357 (2005)
- Borkham-Kamphorst et al., Lab. Invest., 84: 766-777 (April 26, 2004)
- Czochra et al., J Hepatol. (May 30, 2006) [Epub ahead of print; www.elsevier.com/locate/jhep]
- DuoSet® IC Protocol, R&D Systems, Inc., Minneapolis, MN (www.mdsystems.com)
- Fleming et al., Cancer Res., 52: 4550-4553 (1992)
- George, D. Semin. Oncol. 28: 27-33 (2001)
- Gollob, Clin Genitourin. Cancer, 4(3): 167-174 (2005)
- Hart et al., Science, 240: 1529-1531 (June 10, 1988)
- Jones and Cross, Cell Mol Life Sci., 61 (23): 2912-23 (Dec 2004)
- Levitski, A., Cytokine Growth Factor Rev. 15(4): 229-35 (Aug 2004)
- MacDonald et al., Nat. Genet. 29: 143-152 (2001)
- Pietras et al., Cancer Res. 62: 5476-5484 (2002)
- Smith et al., J. Neuropathol. Exp. Neurol. 59: 495-503 (2000)
- Tiesman and Hart, Journal of Biological Chemisty, 268 (13): 9621-9628 (May 5, 1993)

### Abbreviations

The following abbreviations are used herein:
- BL -: bioluminescent
- BSA -: bovine serum albumin
- °C -: degrees centigrade
- Ca -: cancer
- CEA -: carcinoembryonic antigen
- CL -: chemiluminescent
- CMPDs -: chronic myeloproliferative diseases
- CMML -: chronic monomyelocytic leukemia
- DFSP -: dermatofibrosarcoma protruberans
- EDTA -: ethylenediaminetetraacetate
- EGFR -: epidermal growth factor receptor
- ELISA -: enzyme-linked immunosorbent assay
- FGF -: fibroblast growth factor
- FGFR -: fibroblast growth factor receptor
- GISTs -: gastrointestinal stromal tumors
- HRP -: horseradish peroxidase
- M -: molar
- Mab -: monoclonal antibody
- MBC -: metastatic breast cancer
- ml -: milliliter
- mM -: millimolar
- N -: normal or number (depending on context)
- ng -: nanogram
- nm -: nanometer
- OD -: optical density
- pg -: picogram
- PBS -: phosphate buffered saline
- PDGF -: platelet derived growth factor
- PDGFR -: platelet derived growth factor receptor
- pNPP -: paranitrophenyl phosphate
- RTK -: receptor tyrosine kinase
- SD -: standard deviation
- TIMP-1 -: tissue inhibitor of metalloproteinase-1
- TMB -: tetramethylbenzidine
- µg -: microgram
- µl -: microliter
- uPA -: urokinase-type plasminogen activator
- VEGF -: vascular endothelial growth factor
- VEGFR -: vascular endothelial growth factor receptor

### DETAILED DESCRIPTION

The present invention is directed to the methods of claims 1, 3 and 7 using quantitative immunoassays that measure serial changes in the levels of total PDGFR-β protein in human body fluids, which assays are useful diagnostically/prognostically to detect or monitor a disease in a human, or to select a therapy for a patient with said disease, wherein said disease is associated with an activated PDGF pathway. As used herein, an "activated PDGF pathway" is defined as a PDGF pathway activated by either overexpression or mutation of PDGFR-β protein, and therefore encompasses upregulated and/or mutationally stimulated PDGF pathways. Further, as used herein, "total PDGFR-β protein" comprises both phosphorylated and unphosphorylated PDGFR-β protein.

Exemplary of preneoplastic/neoplastic diseases associated with an activated PDGF pathway are the following, as well as precancers leading to the following: metastatic medulloblastoma, GISTs, DFSP, CMPDs, colorectal cancer, colon cancer, lung cancer, non-small-cell lung cancer, small-cell lung cancer, acute myelogenous leukemia, thyroid cancer, pancreatic cancer, bladder cancer, kidney cancer, melanoma, breast cancer, prostate cancer, ovarian cancer, cervical cancer, head-and-neck cancer, brain tumors, hepatocellular carcinoma and hematologic malignancies. In particular, the levels of total PDGFR-β protein, alone or in combination with levels of other proteins, particularly other oncoproteins, can be used to predict clinical outcome and/or as an aid in therapy selection.

The assays of this invention can be diagnostic and/or prognostic, i.e., diagnostic/prognostic. The term "diagnostic/ prognostic" is herein defined to encompass the following processes either individually or cumulatively depending upon the clinical context: determining the presence of disease, determining the nature of a disease, distinguishing one disease from another, forecasting as to the probable outcome of a disease state, determining the prospect as to recovery from a disease as indicated by the nature and symptoms of a case, monitoring the disease status of a patient, monitoring a patient for recurrence of disease, and/or determining the preferred therapeutic regimen for a patient The diagnostic/prognostic methods of this invention are useful, for example, for screening populations for the presence of disease, determining the risk of developing disease, diagnosing the presence of disease, monitoring the disease status of patients, and/or determining the prognosis for the course of disease.

The present invention is useful for screening for the presence of a wide variety of diseases, both non-neoplastic and preneoplastic/neoplastic, as indicated above. Such an assay can be used to detect tumors, monitor their growth, and help in the diagnosis and prognosis of disease. The assays can also be used to detect the presence of cancer metastasis, as well as confirm the absence of tumor tissue following cancer chemotherapy and/or radiation therapy. It can further be used to monitor cancer chemotherapy and tumor reappearance.

The methods include quantifying total PDGFR-β protein, if any, present in serial body fluid samples taken from a human subject diagnosed with, or suspected of having, a preneoplastic/neoplastic disease. The quantified total PDGFR-β protein levels are compared with the average levels in body fluid samples taken from individuals of a control population, wherein an above average level of total PDGFR-β protein is indicative of an activated PDGF pathway. As used herein, an "above average" level of total PDGFR-β protein indicates a level higher than two standard deviations (SD) above the mean level found in control samples.

As used herein, "cancerous" and "neoplastic" have equivalent meanings, and "precancerous" and "preneoplastic" have equivalent meanings. The use of detection of gene expression products as diagnostic/prognostic indicators for diseases is considered conventional by those of skill in the art. However, the application of such approaches to measure quantitatively serial changes in PDGFR-β levels in body fluids, particularly of circulating PDGFR-β levels, to detect or monitor a preneoplastic/neoplastic disease, wherein said disease is associated with an activated PDGF pathway, is new.

### Serial Samples of Body Fluids

In a preferred embodiment of the invention, total PDGFR-β protein is quantitated in human body fluid samples drawn serially over time. The body fluid samples are serum or plasma. Preferred body fluids, for example, include serum, or plasma samples treated with heparin, citrate or EDTA, among other body fluid samples, and can be fresh or frozen. Such body fluid specimens can be taken pretreatment, during treatment, or post-treatment, or can be taken from a patient who is not responding to therapy. As used herein, "serial changes over time" or "serial samples" denotes sequential testing of samples taken over time periods which would be considered relevant for the subject, depending on the context and the circumstances. For example, for cancer patient screening, serial samples might be drawn upon a patient's initial visit, after diagnosis, pre-surgery and/or post-surgery; whereas for population screening for a preneoplastic disease, serial samples might be drawn on a yearly basis.

### Immunoassays

An exemplary and preferred immunoassay according to the methods of the invention is a sandwich ELISA described below in the Materials and Methods section, and was used to obtain the data for Examples 1 through 6. It can be appreciated that alternate methods, in addition to those disclosed herein, can be used to quantify total PDGFR-β protein in human body fluids. Other preferred sandwich assays could be used with other visualizing means, such as luminescent labels. Other labels are detailed below under the subsection labeled Labels.

Many formats can be adapted for use with the methods of the present invention. The detection and quantitation of total PDGFR-β protein in human body fluids can be performed, for example, by enzyme-linked immunosorbent assays, radioimmunoassays, dual antibody sandwich assays, agglutination assays, fluorescent immunoassays, immunoelectron and scanning microscopy using immunogold, among other assays commonly known in the art. The quantitation of total PDGFR-β protein in such assays can be adapted by conventional methods known in the art. In preferred embodiments, serial changes in circulating total PDGFR-β protein levels, or such levels in other body fluids, are detected and quantified by a sandwich assay in which the capture antibody has been immobilized, using conventional techniques, on the surface of the support.

Suitable supports used in assays include among other supports, synthetic polymer supports, such as polypropylene, polystyrene, substituted polystyrene, polyacrylamides (such as polyamides and polyvinylchloride), glass beads, agarose, and nitrocellulose, among other supports.

### Exemplary Immunoassay

An exemplary and preferred ELISA sandwich immunoassay is described in the Materials and Methods section and In Examples 1-6. That exemplary ELISA uses purified mouse anti-human PDGFR-β monoclonal antibody as the capture antibody and blotinylated goat anti-human PDGFR-β polyclonal antibody as the detector antibody. The capture Mab is immobilized on microtiter plate wells; diluted human serum/plasma samples or PDGFR-β standards (recombinant wild-type PDGFR-β protein) are incubated in the wells to allow binding of total PDGFR-β antigen by the capture Mab. After washing of wells, the immobilized PDGFR-β antigen is exposed to a blotinylated detector antibody after which the wells are again washed. A streptavidin-horseradish peroxidase conjugate is then added. After a final wash, TMB Blue Substrate is added to the wells to detect bound peroxidase activity. The reaction is stopped by the addition of 2.5N sulfuric acid, and the absorbance is measured at 450 nm. Correlating the absorbance values of samples with the PDGFR-β standards allows the determination of a quantitative value of total PDGFR-β in pg/ml of serum or plasma.

In an alternate exemplary and preferred embodiment of the invention, the ELISA sandwich Immunoassay comprises the use of a mouse anti-human PDGFR-β monoclonal antibody, AC5.1.3, as the capture antibody and a rabbit anti-human PDGFR-β polyclonal antibody [Austral Biologicals; San Ramon, CA (USA)] as the detector antibody. The AC5.1.3 Mab was prepared against recombinant wild-type total PDGFR-β using standard Kohler-Milstein technologies. In that exemplary immunoassay, the detection means may comprise the use of a horseradish peroxidase-labeled secondary antibody conjugate, e.g., a horseradish peroxidase-labelled goat anti-rabbit conjugate. Alternatively, the rabbit polyclonal detector antibody may be biotinylated prior to binding with strepavidin-horseradish peroxidase, or may be conjugated directly with horseradish peroxidase.

### Combination Assays: Levels of PDGFR-β and Other Protein(s)

It can be appreciated that other proteins, such as inhibitors, oncoproteins, growth factor receptors, angiogenic factors, metastasis proteins, tumor markers, and tumor suppressors, particularly those associated with the PDGF pathway, may be suitable for detection and quantitation along with PDGFR-β, depending on the subject disease. Exemplary and preferred other proteins suitable for testing along with PDGFR-β include tissue inhibitor of metalloproteinase-1 (TIMP-1), HER-2/neu, ras p21, epidermal growth factor receptor (EGFR), platelet derived growth factor receptor alpha (PDGFR-α), vascular endothelial growth factor (VEGF), urokinase-type plasminogen activator (uPA), carcinoembryonic antigen (CEA), and p53. Assays to detect preferred other proteins suitable for the methods of the invention are known to one of skill in the art. For example, immunoassays for the quantitation of HER-2/neu and TIMP-1 in human body fluids are commercially available, such as the Oncogene Science TIMP-1 ELISA [Oncogene Science, Cambridge, MA (USA), www.oncogene.com], which can determine ng/ml values of TIMP-1 levels in human serum or plasma.

### Prognosis

Monitoring the levels of PDGFR-β and/or other proteins can be indicative of clinical outcomes for diseases. A preferred method of evaluating a clinical outcome is selected from the group consisting of response rate (RR), complete response (CR), partial response (PR), stable disease (SD), clinical benefit [including complete response (CR), partial response (PR), and stable disease (SD)], time to progression (TTP), progression free survival (PFS) and overall survival (OS).

### Antibodies

The term "antibody" herein is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity. Antibodies useful according to the methods of the invention may be prepared by conventional methodology and/or by genetic engineering. Antibodies according to the invention are those that bind to "total PDGFR-β", (both tyrosine-phosphorylated and unphosphorylated PDGFR-β). Most preferred are antibodies that selectively bind the ectodomain of PDGFR-β.

"Antibody fragments" comprise a portion of a full length antibody, generally the antigen binding or variable domain thereof. Examples of antibody fragments include Fab, Fab', F(ab')₂, and Fv fragments; diabodies; linear antibodies; single-chain antibody molecules; bispecific antibodies; and multispecific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity [U.S. Pat. No. 4,816,567; and Morrison et al., Proc. Natl. Acad. Sci., USA, 81: 6851-6855 (1984)].

"Humanized" forms of non-human (e.g., murine) antibodies are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or nonhuman primate having the desired specificity, affinity, and capacity. In some Instances, framework region (FR) residues of the human immunoglobulin are replaced by corresponding non-human residues. Furthermore, humanized antibodies may comprise residues which are not found in the recipient antibody or in the donor antibody. Such modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature, 321:522-525 (1986); Reichmann et al., Nature, 332: 323-329 (1988); and Presta, Curr. Op. Struct. Biol., 2: 593-596 (1992).

"Single-chain Fv" or "sFv" antibody fragments comprise the V_{H} and V_{L} domains of antibody, wherein these domains are present in a single polypeptide chain. Generally, the Fv polypeptide further comprises a polypeptide linker between the V_{H} and V_{L} domains which enables the sFv to form the desired structure for antigen binding. For a review of sFv see Pluckthun in The Pharmacology of Monoclonal Antibodies, Vol. 113, Rosenburg and Moore eds. Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci., USA, 90: 6444-6448 (1993).

The expression "linear antibodies" refers to the antibodies described in Zapata et al., Protein Eng., 8(10): 1057-1062 (1995). Briefly, such antibodies comprise a pair of tandem Fd segments (V_{H}-C_{H}1-V_{H}-C_{H}1) which form a pair of antigen binding regions. Linear antibodies can be bispecific or monospecific.

Representative monoclonal antibodies useful according to this invention include mouse anti-human total PDGFR-β Mabs , such as those found in the R&D Systems sandwich ELISA kit designed to measure human total PDGFR-β in cell lysates [DuoSet^{®} IC, Catalog # DYC385-5]. Monoclonal antibodies useful according to this invention serve to identify total PDGFR-β proteins in various laboratory prognostic tests, for example, in clinical samples. A preferred monoclonal antibody for use in the invention is the mouse anti-human PDGFR-β monoclonal antibody, AC5.1.3, which was prepared against recombinant wild-type human total PDGFR-β.

General texts describing additional molecular biological techniques useful herein, including the preparation of antibodies include Berger and Kimmel, Guide to Molecular Cloning Techniques, Methods in Enzymology, Vol. 152, Academic Press, Inc., Sambrook et al., Molecular Cloning: A Laboratory Manual, (Second Edition, Cold Spring Harbor Laboratory Press; Cold Spring Harbor, N.Y.; 1989) Vol. 1-3; Current Protocols in Molecular Biology, F. M. Ausabel et al. [Eds.], Current Protocols, a joint venture between Green Publishing Associates, Inc. and John Wiley & Sons, Inc. (supplemented through 2000), Harlow et al., Monoclonal Antibodies: A-Laboratory Manual, Cold Spring Harbor Laboratory Press (1988), Paul [Ed.]; Fundamental immunology, Lippincott Williams & Wilkins (1998), and Harlow et al., Using Antibodies: A Laboratory Manual, Cold Spring Harbor Laboratory Press (1998).

### Labels

The antibodies useful according to this invention to identify total PDGFR-β proteins can be labeled in any conventional manner. A preferred label, according to this invention is horseradish peroxidase, and a preferred method of labeling the antibodies is by using biotin-strepavidin complexes.

As appropriate, antibodies used in the immunoassays of this invention that are used as tracers may be labeled in any manner, directly or indirectly, that results in a signal that is visible or can be rendered visible. Detectable marker substances include radionuclides, such as ³H, ¹²⁵I, and ¹³¹I; fluorescers, such as, fluorescein isothiocyanate and other fluorochromes, phycobiliproteins, phycoerythin, rare earth chelates, Texas red, dansyl and rhodamine; colorimetric reagents (chromogens); electron-opaque materials, such as colloidal gold; bioluminescers; chemiluminescers; dyes; enzymes, such as, horseradish peroxidase, alkaline phosphatases, glucose oxidase, glucose-6-phosphate dehydrogenase, acetylcholinesterase, alpha -, beta-galactosidase, among others; coenzymes; enzyme substrates; enzyme cofactors; enzyme inhibitors; enzyme subunits; metal ions; free radicals; or any other immunologically active or inert substance which provides a means of detecting or measuring the presence or amount of immunocomplex formed. Exemplary of enzyme substrate combinations are horseradish peroxidase and tetramethyl benzidine (TMB), and alkaline phosphatases and paranitrophenyl phosphate (pNPP).

Another preferred detection, or detection and quantitation systems according to this invention produce luminescent signals, bioluminescent (BL) or chemiluminescent (CL). In chemiluminescent (CL) or bioluminescent (BL) assays, the intensity or the total light emission is measured and related to the concentration of the unknown analyte. Light can be measured quantitatively using a luminometer (photomultiplier tube as the detector) or charge-coupled device, or qualitatively by means of photographic or X-ray film. The main advantages of using such assays is their simplicity and analytical sensitivity, enabling the detection and/or quantitation of very small amounts of analyte.

Exemplary luminescent labels are acridinium esters, acridinium sulfonyl carboxamides, luminol, umbelliferone, isoluminol derivatives, photoproteins, such as aequorin, and luciferases from fireflies, marine bacteria, Vargulla and Renilla. Luminol can be used optionally with an enhancer molecule, preferably selected from the group consisting of 4-iodophenol or 4-hydroxy-cinnamic acid. Acridinium esters are one of the preferred types of CL labels according to this invention. Typically, a CL signal is generated by treatment with an oxidant under basic conditions.

Also preferred luminescent detection systems are those wherein the signal (detectable marker) is produced by an enzymatic reaction upon a substrate. CL and BL detection schemes have been developed for assaying alkaline phosphatases (AP), glucose oxidase, glucose 6-phosphate dehydrogenase, horseradish peroxidase (HRP), and xanthine-oxidase-labeis, among others. AP and HRP are two preferred enzyme labels which can be quantitated by a range of CL and BL reactions. For example, AP can be used with a substrate, such as an adamantyl 1,2-dioxetane aryl phosphate substrate (e.g. AMPPD or CSPD; [Kricka, L.J., "Chemiluminescence and Bioluminescence, Analysis by," at p. 167, Molecular Biology and Biotechnology: A Comprehensive Desk Reference (ed. R.A. Meyers) (VCH Publishers; N.Y., N.Y.; 1995)]; preferably a disodium salt of 4-methoxy-4-(3-phosphatephenyl) spiro [1,2-dioxetane-3,2'-adamantane], with or without an enhancer molecule, preferably, 1-(trioctylphosphonium methyl)-4- (tributylphosphonium methyl) benzene diochloride. HRP is preferably used with substrates, such as, 2',3',6'-trifluorophenyl-methoxy-10-methylacridan-9-carboxylate.

CL and BL reactions can be adapted for analysis not only of enzymes, but also of other substrates, cofactors, inhibitors, metal ions and the like. For example, luminol, firefly luciferase, and marine bacterial luciferase reactions are indicator reactions for the production or consumption of peroxide, ATP, and NADPH, respectively. They can be coupled to other reactions involving oxidases, kinases, and dehydrogenases, and can be used to measure any component of the coupled reaction (enzyme, substrate, cofactor).

The detectable marker may be directly or indirectly linked to an antibody used in an assay of this invention. Exemplary of an indirect linkage of the detectable label is the use of a binding pair between an antibody and a marker or the use of a signal amplification system.

Exemplary of binding pairs that can be used to link antibodies of assays of this invention to detectable markers are biotin/avidin, streptavidin, or anti-blotin; avidin/anti-avidin; thyroxine/thyroxine-binding globulin; antigen/antibody; antibody/ anti-antibody; carbohydrate/lectins; hapten/anti-hapten antibody; dyes and hydrophobic molecules/hydrophobic protein binding sites; enzyme inhibitor, coenzyme or cofactor/enzyme; polynucleic acid/homologous polynucleic acid sequence; fluorescein/anti- fluorescein; dinitrophenol/anti-dinitrophenol; vitamin B12/intrinsic factor; cortisone, cortisol/cortisol binding protein; and ligands for specific receptor protein/membrane associated specific receptor proteins. Preferred binding pairs according to this invention are biotin/avidin or streptavidin, more preferably biotin/ streptavidin.

Various means for linking labels directly or indirectly to antibodies are known in the art. For example, labels may be bound either covalently or non-covalently. Exemplary antibody conjugation methods are described in: Avarmeas et al., Scan. J. Immunol., 8 (Suppl. 7): 7 (1978); Bayer et al., Meth. Enzymol., 62: 308 (1979); Chandler et al., J. Immunol. Meth., 53: 187 (1982); Ekeke and Abuknesha, J. Steroid Biochem., 11: 1579 (1979); Engvall and Perlmann, J. Immunol., 109: 129 (1972); Geoghegan et al., Immunol. Comm., 7: 1 (1978); and Wilson and Nakane, Immunofluorescence and Related Techniques, p. 215 [Elsevier/North Holland Biomedical Press; Amsterdam (1978)].

Depending upon the nature of the label, various techniques can be employed for detecting and quantitating the label. For fluorescers, a large number of fluorometers are available. For chemiluminescers, luminometers or films are available. With enzymes, a fluorescent, chemiluminescent, or colored product can be determined or measured fluorometrically, luminometrically, spectrophotometrically or visually.

Various types of chemiluminescent compounds having an acridinium, benzacridinium, or acridan type of heterocyclic ring systems are preferred labels. Acridinium and benzacridinium esters are currently the more preferred chemiluminescent compounds, with preferred acridinium esters including those compounds having heterocyclic rings or ring systems that contain the heteroatom in a positive oxidation state including such ring systems as acridinium, benz[a]acridinium, benz[b]acridinium, benz[c]acridinium, a benzimidazole cation, quinolinium, isoquinolinium, quinolizinium, a cyclic substituted quinolinium, phenanthridinium, and quinoxalinium, as are well-known in the art.

The tracer may be prepared by attaching to the selected antibody either directly or indirectly a reactive functional group present on the acridinium or benzacridinium ester, as Is well known to those skilled in the art, e.g. Weeks et al., Clinical Chemistry, 29(8), 1474-1479, (1983). Particularly preferred compounds are acridinium and benzacridinium esters with an aryl ring leaving group and the reactive functional group present in either the para or the meta position of the aryl ring. [See, U.S. Patent No. 4,745,181 and WO 94/21823.]

### PDGF Pathway-directed Therapies

As used herein, "PDGF pathway-directed therapies" include any therapies that are targeted to the PDGF pathway, including inhibition of PDGFR protein expression (e.g., antisense oligonucleotides), prevention of membrane localization essential for PDGFR activation, or inhibition of downstream effectors of PDGFR (e.g., Raf serine/threonine kinases). PDGF pathway-directed therapies may be multi-kinase Inhibitors, tyrosine kinase inhibitors, monoclonal antibodies, and bis-aryl ureas.

An exemplary and preferred tyrosine kinase inhibitor is imatinib mesylate [also known as STI571 or Gleevec®; Novartis Pharma AG (E. Hanover, N.J., USA and Basel, Switzerland)]. Another preferred PDGF pathway-directed therapy according to the Invention is the bis-aryl urea Sorafenib (BAY 43-9006) [Onyx Pharmaceuticals, Richmond, CA (USA), and Bayer Corporation, West Haven, CT (USA); Lyons et al., Endocrine-Related Cancer, 8: 219-225 (2001) and Wilhelm et al. (2004)], a small molecule and novel dual-action inhibitor of both Raf (a protein-serine/threonine kinase) and VEGFR (vascular endothelial growth factor receptor, a receptor tyrosine kinase), and consequently an Inhibitor of both tumor cell proliferation and angiogenesis. In addition, Sorafenib has been found to inhibit several other receptor tyrosine kinases involved in tumor progression and neovascularization, including PDGFR-β, Flt-3, and c-KIT. PD166285 [Pfizer (www.pfizer.com)], a general tyrosine kinase Inhibitor, can antagonize both PDGF and FGF-2-mediated responses. [Bansal et al., J. Neuroscience Res., 74(4): 486-493 (published online 23 Oct. 2003).]

Other exemplary therapies that target the PDGF pathway include: Sutent/SU11248, PTK 787, MLN518, PKC-412, CDP860, ABT-869, AMG 706 and XL9999. Sutent/SU11248 (sunitinib malate; an indoline-2-one) [Pfizer (www.pfizer.com)] targets receptor tyrosine kinases (RTKs) including PDGFR, with anti-angiogenic and anti-tumor effects. PDGFR plays a significant role in fostering angiogenesis by regulating the proliferation and migration of pericytes, cells that support blood vessels, and Sutent/SU11248 is believed to inhibit PDGFR's angiogenic action.

PTK 787 [Novartis, supra, and Schering AG (Berlin, Germany)] is a oral small molecule anti-angiogenesis agent (anilinophthalazine) active against PDGFR, as well as against VEGFR and c-Kit tyrosine kinase receptors. [See, for example, Garcia-Echevera and Fabbro, "Therapeutically Targeted Anti-cancer Agents: Inhibition of Receptor Tyrosine Kinases," Mini Reviews in Medicinal Chemistry, 4(3): 273-283 (March 2004).]

MLN518 [formerly known as CT53518; Millenium Pharmaceuticals (www.mlnm.com); Cambridge, MA (USA)] is an oral, small molecule designed to inhibit type III receptor tyrosine kinases (RTKs), including PDGFR, FLT3 and c-Kit.

PKC-412 [midostaurin; N-benzoyl-staurosporine (a derivative of staurosporine, a product of Streptomyces bacteria); Novartis, supra] inhibits PDGFR, VEGFR and multiple protein kinase Cs, "which makes it especially attractive in patients with wild-type KIT with mutations in PDGFR." [PKC 412-An Interview with Charles Blanke, MD, FACP (www.gistsupport.org/pkc412.html); see also Reichardt et al., J. Clin. Oncol., 23(16S): 3016 (June 1 Suppl., 2005).]

CDP860 [anti-PDGFR-beta antibody fragment; Celltech (Slough, UK)] is an engineered Fab' fragment-polyethylene glycol conjugate, which binds to and blocks the activity of the beta-subunit of PDGFR. [Jayson et al., J. Clin. Oncol., 23(5): 973-981 (Feb. 10, 2005), which suggests in its abstract "that inhibition of PDGFR-beta might improve delivery of concurrently administered therapy."]

ABT-869 [Abbott Laboratories, Abbott Park, Illinois (USA)] is similar in activity to SU11248, being a multitargeted RTK inhibitor of the VEGF and PDGF receptor families. In preclinical tests, the IC₅₀ for PDGFR-β was 2 nanomolar [Albert et al., Mol Cancer Ther., 5(4): 995-1006 (2006)].

AMG 706 [Amgen Inc. Thousand Oaks, California (USA)] is an oral, multi-kinase inhibitor of VEGF receptors, PDGF receptor, and Kit. Clinical trials evaluating AMG 706 monotherapy in advanced thyroid cancer and in imatinib-resistant GIST are ongoing.

XL999 [one of several Spectrum Selective Kinase Inhibitors^{™} (SSKIs) from Exelixis (South San Francisco, CA, USA)] Inhibits PDGFR-beta, as well as other RTKs, such as VEGFR, FGFR1 and FLT3.

### Therapies Directed to Crosstalk Pathways

It can be appreciated that other therapies may be useful according to the methods of the invention, such as therapies that specifically target pathways that crosstalk with the PDGF/PDGFR pathway. For example, Saito et al. [Mol. Cell Biol. 21(19): 6387-6394 (2001)] has reported crosstalk between PDGFR-β and EGFR, and the formation of PDGFR-β-EGFR heterodimers. Drugs that target the EGFR pathway may therefore be useful to treat PDGFR pathway-related diseases. Exemplary EGFR-targeted therapies that may be useful according to the invention include the following: Iressa® [AstraZeneca, Wilmington, DE (USA), www.astrazeneca-us.com], Tarceva® [OSI Pharmaceuticals, Melville, NY (USA), www.osip.com], Erbitux® [Bristol-Myers Squibb, Princeton, NJ (USA)], ABX-EGF [Abgenix, Fremont, CA (USA)], AEE788 [Novartis, E. Hanover, N.J.(USA), and Basel, Switzerland], AG1478 [Calbiochem, San Diego, CA (USA)], DAB389EGF [Saragen, Boston, MA (USA)], EMD72000 [Merck KGaA, Darmstadt, Germany], MDX-214 and MDX-447 [Medarex, Annandale, NJ (USA)], PD166285 and PD153055 [Pfizer (www.pfizer.com)].

### EXAMPLES

The following examples are for purposes of illustration only and are not meant to limit the invention in any way.

### Materials and Methods

### Solid Phase Sandwich Microtiter ELISA for Human Serum and Plasma Sample Preparation

Suitable samples for analysis by the PDGFR-β ELISA include human plasma treated with heparin, citrate, or EDTA, and human serum. Due to possible interfering factors, special care must be taken in the preparation and assay of human serum and plasma. Any flocculant material should be removed from samples by microcentrifugation prior to dilution. The initial concentration of the serum or plasma specimen to be examined should be about 12-13% (a 1:8 dilution of specimen in sample diluent). For example, 40 µl of sample may be diluted into 280 µl of sample diluent, and 100 µl added to the microplate wells.

### Exemplary Assay Procedure

The following ELISA protocol is a modified ELISA using the basic components from the R&D Systems DuoSef^{®} IC [Catalog # DYC385-5] for a sandwich ELISA to measure human total PDGFR-β in cell lysates.

Purified mouse anti-human PDGFR-β monoclonal antibodies [R&D Systems Part #841502] are diluted to 4 µg/ml in PBS [Sigma] and are adsorbed to wells (at 100 µl per well) of 96-well microplates [Nunc MaxiSorp^{™} F8 Immuno Module, Part #1001; Nalge Nunc International Corp., Naperville, IL (USA)]. Adsorption is carried out overnight (at least 12 hours) at room temperature (20°-27°C). At the end of the incubation period, blocking solution is added to the wells (0.002M sodium phosphate monobasic, 0.008M sodium phosphate dibasic heptahydrate, 0.15M sodium chloride, 0.29M beta D-lactose and 2% BSA reagent grade; pH 7.45) and aspirated using an Oyster Bay Microplate Coater (Model MPCS-3) [Oyster Bay Pump Works, Hicksville, N.Y. (USA)]. Coated microplates are dried using a LyoStar® Freeze-Dryer [FTS Systems, Stone Ridge, N.Y. (USA)] at 22°C and stored at 2-8°C.

Human serum or plasma samples and PDGFR-β standards [R&D Systems, Inc., Part #841504; Minneapolis, MN (USA)] are diluted with sample diluent [4% BSA Protease Free (Serologicals Product #82-045; www.serologicals.com), 10 µg/ml Purified Mouse IgG (Scantibody Laboratories Product #3BM222; Santel, CA, USA) and 0.09% sodium azide]. Plasma samples treated with heparin, citrate or EDTA may be used instead of serum. Diluted samples are then added to microplate wells (100 µl per well) and the sealed plates incubated for 2 hours in a room temperature incubator. Each well is washed six times with 300 µL per well of platewash, pH 7.4 [0.137M sodium chloride, 2.7mM potassium chloride, 1.45 mM potassium phosphate monobasic, 0.08 M sodium phosphate dibasic heptahydrate, and 0.05% Tween 20]. To each well is then added 100 µl of biotinylated Goat anti-human PDGFR-β polyclonal antibody [R&D Systems, Inc., Part #841503; Minneapolis, MN (USA)] diluted to 100 ng/ml in detector diluent [0.4% casein, 1.7mM sodium phosphate monobasic, 8.1 mM sodium phosphate dibasic heptahydrate, 0.15M sodium chloride, and 0.1% sodium azide, pH 7.15±0.1].

The plates are incubated for 2 hours in a room temperature incubator. Each well is washed six times as above, and 100 µl of Strepavidin-HRP [R&D Systems Part #890803] diluted 1:200 in conjugate diluent [1.7mM sodium phosphate monobasic, 8.1 mM sodium phosphate dibasic heptahydrate, 0.145M sodium chloride, 0.1% 2-chloroacetamide, 1% BSA Protease Free, and 0.05% Tween 20; pH 7.3] is added to each well. The plates are incubated in a room temperature incubator for 20 minutes. Each well is again washed six times as above, and 100 µl of a color producing substrate solution [TMB Blue Substrate, DakoCytomation, Catalog # S1601; www.dakocytomation.com] is added to each well to detect bound peroxidase activity. The plates are incubated 30 minutes in a room temperature incubator, and the reaction is stopped by the addition of 100 µl 2.5N sulfuric acid. The absorbance is measured at 450 nm using a Molecular Devices spectrophotometric plate reader (Model #Vmax; www.moleculardevices.com) and analyzed using a Softmax Pro^{™} (Molecular Devices Corporation; www.moleculardevices.com) software program.

### Standard Curves

Quantitative analyses were made by constructing a standard curve using PDGFR-β standard [recombinant human PDGFR-β from R&D Systems, Part # 841504] at 7 different concentrations of 125, 250, 500, 1000, 2000, 4000, and 8000 pg/ml.

### Competition Assay

In order to confirm that the ELISA was specific to PDGFR-p, human serum and plasma samples were tested in a competition assay. Either excess free capture Mab [anti-PDGFR-β Mab, R&D Systems Part #841502] or an irrelevant control Mab MOPC21 [Sigma Product #M9269; purified Mouse IgGI, kappa from murine myeloma] were preincubated [at a final concentration of 8 µg/ml, or 2X coating concentration of 4µg/ml] with the samples (final dilution 1:10) for 30 minutes at 37°C, before assaying 100 µl of the pretreated samples in plate wells coated with capture Mabs, and processed as described above.

### Human Serum and Plasma Samples

Frozen plasma and serum samples from normal humans and cancer patients were obtained from Bioclinical Partners, Inc. (now Zeptometrix) [Franklin, MA (USA)].

### Example 1

### Normal Human Serum

Microplate wells were coated with anti-total PDGFR-β capture antibody, as described above. A total of 12 normal human sera (4 female, 8 male; obtained from Bioclinical Partners, Inc.) were individually tested in the coated microplates by ELISA, and PDGFR-β present in the sera was detected using biotinylated goat anti-human PDGFR-β polyclonal antibody [R&D Systems Part #841503]. The absorbance index at 450 nm was used to determine the level of total PDGFR-β in serum samples. The results show that, in general, similar levels of total PDGFR-β protein were found in normal females and normal males (average of 29 ng/ml in females vs. 28 ng/ml in males).

**Table 1: Serum Samples from Normal Humans**

| **Normal Female** | | **Normal Male** | |
|---|---|---|---|
| **Serum Samples** | | **Serum Samples** | |
| **Sample** | **PDGFR-β** **(pg/mL)** | **Sample** | **PDGFR-β** **(pg/mL)** |
| 4097 | 31951.82 | 4162 | 31548.96 |
| 4098 | 33064.59 | 4163 | 27008.16 |
| 4099 | 28650.47 | 4164 | 31918.65 |
| 4100 | 23851.61 | 4165 | 20326.66 |
| | | 1078 | 32336.34 |
| | | 1079 | 24555.23 |
| | | 1080 | 24519.42 |
| | | 1083 | 33075.68 |
| | | | |
| **Mean** | **29379.6** | **Mean** | **28161.1** |
| **SD** | **3580.7** | **SD** | **4418.1** |

### Example 2

### Breast Cancer Serum and Plasma

Eight breast carcinoma samples (seven plasma, one late stage serum) were assayed for total PDGFR-β protein levels by ELISA, as described under Materials and Methods. The results are shown In Table 2 below.

**Table 2**

| Breast Cancer Patient Samples (PDGFR-β Levels) | |
|---|---|
| **Breast Cancer** | |
| **Sample** | **PDGFR-β** **(pg/mL)** |
| **Plasma** | |
| 2113 | 31148.55 |
| 2114 | 39448.79 |
| 2116 | 33684.12 |
| 2118 | 38213.55 |
| 2119 | 35234.52 |
| 2120 | 29941.98 |
| 2121 | 26614.88 |
| | |
| **Mean** | **33469.5** |
| **SD** | **4586.9** |
| | |
| **Late Stage Serum** | |
| 2689 | 20005.84 |

Pretreatment elevated levels of the circulating PDGFR-β protein in metastatic breast cancer (MBC) patients may indicate those patients eligible for either conventional or targeted anti-PDGFR therapies.

In addition, increasing or decreasing levels of PDGFR-β alone, or in conjunction with levels of HER-2/neu, in breast cancer patients may be an early indicator of recurrent breast cancer. Pretreatment elevated circulating levels of PDGFR-β or HER-2/neu in metastatic breast cancer (MBC) patients may indicate those patients eligible for anti-PDGFR and/or anti-HER-2/neu therapies, either conventional or targeted therapies.

### Example 3

### Ovarian Cancer and Benign Disease Serum

Serum samples from patients with either benign ovarian disease (five patients) or ovarian carcinoma (three patients) were assayed by ELISA for total PDGFR-β (in all instances, using mouse anti-human PDGFR-β monoclonal antibody as the capture antibody and biotinylated goat anti-human PDGFR-β polyclonal antibody as the detector antibody, as described in Materials and Methods).

**Table 3**

| Ovarian Disease Patient Samples (PDGFR-β Levels) | | | |
|---|---|---|---|
| **Benign** **Sample** | **PDGFR-β** **(pg/mL)** | **Cancer** **Sample** | **PDGFR-β** **(pg/mL)** |
| **2362** | 19361.25 | **2347** | 27317.23 |
| **2363** | 21299.72 | **2349** | 29484.69 |
| **2364** | 24738.85 | **2352** | 25453.79 |
| **2365** | 28625.58 | | |
| **2366** | 27449.30 | | |
| | | | |
| **Mean** | **24294.9** | **Mean** | **27418.8** |
| **SD** | **3527.4** | **SD** | **2017.4** |

### Example 4

### PDGFR-β Levels in Prostate Cancer Plasma

Plasma samples from eleven prostate cancer patients were assayed by ELISA for total PDGFR-β levels as described above under Materials and Methods (Table 4).

**Table 4**

| Plasma Samples from Prostate Cancer Patients (PDGFR-β Levels) | |
|---|---|
| **Sample** | **PDGFR-β** **(pg/mL)** |
| **2254** | 28855.00 |
| **2255** | 26602.41 |
| **2256** | 41338.89 |
| **2257** | 32275.70 |
| **2258** | 15595.52 |
| **2259** | 30020.62 |
| **2260** | 28543.51 |
| **2261** | 27960.64 |
| **2347** | 27317.23 |
| **2349** | 29484.69 |
| **2352** | 25453.79 |
| **Mean** | **28495.3** |
| **SD** | **5749.29** |

### Example 5

### PDGFR-β Levels in Colon Cancer Serum

Based on the hypothesis of the invention, that a PDGF pathway activated by either phosphorylation or overexpression of PDGFR-β may result in elevated levels of total PDGFR-β protein in body fluids, three sera from active colon cancer patients were assayed for total PDGFR-β levels (Table 5).

**Table 5**

| Colon Cancer Patient Serum Samples (Total PDGFR-β Levels) | |
|---|---|
| **Sample** | **PDGFR-β** **(pg/mL)** |
| 2779 | 16458.99 |
| 2784 | 33316.96 |
| 2785 | 23816.68 |
| **Mean** | **24530.9** |
| **SD** | **8451.6** |

Increasing levels of PDGFR-β may be indicative of colon cancer recurrence or progression and identify patients who should be treated with either conventional therapies or PDGF pathway targeted therapies. Increasing levels of PDGFR-β may be used in conjunction with other oncology tests, such as carcinoembryonic antigen (CEA).

### Example 6

### Specificity: Competition Assay

In order to confirm that the ELISA used in Examples 1-5 was specific to PDGFR-β, human serum and plasma samples were tested in a competition assay, in which either excess free capture Mab [anti-PDGFR-β Mab, R&D Systems] or an irrelevant control Mab MOPC21 were preincubated with the samples for 30 minutes at 37°C, before assaying the samples on plates coated with capture Mabs, as described in the ELISA protocol above under Materials and Methods.

**Table 6**

| Human Serum Samples (PDGFR-β Levels) | | | |
|---|---|---|---|
| **Source** | **Sample Pretreatment** **(1:10 dII.)** | **PDGFR-β** **(pg/mL)** | **% Inhibition** |
| Breast Ca plasma | 2118 | 46913.98 | N/A |
| | 2118+Capture Mab | 1578.69 | 96.6 |
| | 2118+MOPC21 | 34569.15 | 26.3 |
| Prostate Ca plasma | 2259 | 29459.06 | N/A |
| | 2259+Capture Mab | 1291.63 | 95.6 |
| | 2259+MOPC21 | 28890.80 | 1.9 |
| Ovarian Ca serum | 2352 | 37325.06 | N/A |
| | 2352+Capture Mab | 1435.52 | 96.2 |
| | 2352+MOPC21 | 32729.97 | 12.3 |
| Normal fem. serum | 4098 | 32433.46 | N/A |
| | 4098+Capture Mab | 1590.27 | 95.1 |
| | 4098+MOPC21 | 32851.44 | -1.3 |
| Normal male serum | 4163 | 24195.74 | N/A |
| | 4163+Capture Mab | 1534.46 | 93.7 |
| | 4163+MOPC21 | 30707.53 | -26.9 |

As shown in Table 6, the ELISA was very specific for PDGFR-β, as in all samples preincubation with the anti-PDGFR-β Mab inhibited capture by about 95%, whereas preincubation with the irrelevant Mab MOPC21 generally did not inhibit capture significantly.

## Claims

1. A method of monitoring the status of a cancerous or precancerous disease associated with upregulation of PDGFRbeta levels in a patient and/or monitoring how a patient with said disease is responding to a therapy, comprising immunologically detecting and quantifying serial changes in total PDGFR-β protein levels in serum or plasma samples taken from said patient over time with antibodies binding total PDGFR-β protein;
wherein increasing levels of total PDGFR-β protein over time indicate disease progression or a negative response to said therapy, and wherein decreasing levels of total PDGFR-β protein over time indicate disease remission or a positive response to said therapy.

2. The method of claim 1, wherein said immunological detection and quantitation is by a sandwich immunoassay.

3. A method of therapy selection for a human patient with a cancerous or precancerous disease associated with upregulation of PDGFRbeta levels , comprising:
(a) immunologically detecting and quantifying the average level of total PDGFR-β protein in serum or plasma samples taken from individuals of a control population with antibodies binding total PDGFR-β protein;
(b) immunologically detecting and quantifying serial changes in total PDGFR-β protein levels in serum or plasma samples taken from the patient over time with antibodies binding total PDGFR-β protein;
(c) comparing the levels of total PDGFR-β protein in the patient's samples to the average level of total PDGFR-β protein in the control samples; and
(d) deciding whether to use conventional therapy and/or PDGF pathway-directed therapy to treat the patient based upon the differences between the levels of total PDGFR-β protein in the patient's samples and the average level of total PDGFR-β protein in the control samples, and in view of the serial changes among the levels of total PDGFR-β protein in the patient's samples,
wherein levels that are trending above the average level of total PDGFR-β protein in the control samples indicate a PDGF driven disease, and the decision may be made to use PDGF pathway-directed therapy to treat the patient.

4. The methods of claims 1, 2 and 3, wherein said therapy is selected from multi-kinase inhibitors, tyrosine kinase inhibitors, monoclonal antibodies, and bis-aryl ureas.

5. The methods of claims 1 , 2 and 3, wherein said therapy is a PDGF pathway-directed therapy.

6. The methods of claim 5, wherein said PDGF pathway-directed therapy is the tyrosine kinase inhibitor imatinib mesylate or the bis-aryl urea Sorafenib (BAY 43-9006).

7. A diagnostic method to detect a cancerous or precancerous disease associated with upregulation of PDGFRbeta levels in a human subject, comprising:
(a) immunologically detecting and quantifying the average level of total PDGFR-β protein in serum or plasma samples taken from individuals of a control population with antibodies binding total PDGFR-β protein;
(b) immunologically detecting and quantifying serial changes in total PDGFR-β protein in serum or plasma samples taken from the subject over time with antibodies binding total PDGFR-β protein; and
(c) comparing the levels of total PDGFR-β protein in the subject's samples to the average level of total PDGFR-β protein in the control samples; wherein a level of total PDGFR-β protein in the subject's samples that is above the average level of total PDGFR-β protein in the control samples is indicative of an activated PDGF pathway and the presence of disease in the subject.

8. The methods of claims 3 and 7, wherein said immunological detection and quantification of steps (a) and (b) are by sandwich immunoassay.

9. The methods of claims 2 and 8, wherein the sandwich immunoassay comprises the use of one or more monoclonal antibodies that selectively bind the ectodomain of PDGFR-β protein.

10. The methods of claim 1, wherein said preneoplastic/neoplastic disease is selected from the group consisting of metastatic medulloblastoma, dermatofibrosarcoma protruberans, gastrointestinal stromal tumors, colorectal cancer, colon cancer, lung cancer, non-small-cell lung cancer, small-cell lung cancer, chronic myeloproliferative diseases, acute myelogenous leukemia, thyroid cancer, pancreatic cancer, bladder cancer, kidney cancer, melanoma, breast cancer, prostate cancer, ovarian cancer, cervical cancer, head-and-neck cancer, brain tumors, hepatocellular carcinoma, hematologic malignancies, and precancers leading to the aforementioned cancers.

11. The methods of claims 1 , 3 and 7 which are further prognostic for said disease, wherein said levels of total PDGFR-β protein in the patient's samples are indicative of a better or poorer prognosis for said patient.

12. The methods of claim 11, wherein said prognosis is a clinical outcome selected from the group consisting of response rate (RR), complete response (CR), partial response (PR), stable disease (SD), time to progression (TTP), progression free survival (PFS), overall survival (OS), and clinical benefit, which comprises complete response (CR), partial response (PR), and stable disease (SD).

13. The methods of claim 11, wherein increasing levels of total PDGFR-β are indicative of a greater probability of early recurrence or metastasis.

14. The methods of claims 1, 3 and 7 wherein said patient's samples are pretreatment samples.

15. The methods of claim 1, 3 and 7 further comprising the use of an immunoassay to detect or detect and quantify levels of one or more other proteins in the subject's samples.

16. The methods of claim 15, wherein said other protein is or said other proteins are selected from the group consisting of inhibitors, oncoproteins, growth factor receptors, angiogenic factors, metastasis proteins, tumor markers, and tumor suppressors.

17. The methods of claim 16 wherein said inhibitor is tissue inhibitor of metalloproteinase-1 (TIMP-1), said oncoproteins are selected from the group consisting of HER-2/neu and ras p21 , said growth factor receptors are selected from the group consisting of epidermal growth factor receptor (EGFR) and platelet derived growth factor receptor alpha (PDGFR-α), said angiogenic factor is vascular endothelial growth factor (VEGF), said metastasis protein is urokinase-type plasminogen activator (uPA), said tumor marker is carcinoembryonic antigen (CEA), and said tumor suppressor is p53.

18. The methods of claim 15, wherein said other protein is PDGFR-α protein.

19. The methods of claims 1 and 3, wherein the body fluid samples are from a cancer patient who has not responded to treatment.

## Patentansprüche

1. Verfahren zum Überwachen des Status einer kanzerösen oder präkanzerösen Krankheit, die mit einer Hinaufregulation der PDGFR-beta-Spiegel einhergeht, in einem Patienten und/oder zum Überwachen, wie ein Patient mit dieser Krankheit auf eine Therapie reagiert, umfassend den immunologischen Nachweis und die Quantifizierung serieller Veränderungen in den Gesamt-PDGFR-β-Protein-Spiegeln in Serum- oder Plasmaproben, die von dem Patienten entnommen wurden, über die Zeit mit Antikörpern, die an Gesamt-PDGFR-β-Protein binden;
wobei ansteigende Spiegel an Gesamt-PDGFR-β-Protein über die Zeit auf ein Voranschreiten der Krankheit oder ein negatives Ansprechen auf die Therapie hinweisen und wobei abnehmende Spiegel an Gesamt-PDGFR-β-Protein über die Zeit auf eine Remission der Krankheit oder ein positives Ansprechen auf die Therapie hinweisen.

2. Verfahren nach Anspruch 1, wobei der immunologische Nachweis und die Quantifizierung mittels Sandwich-Immunassay erfolgen.

3. Verfahren zum Auswählen einer Therapie für einen menschlichen Patienten mit einer kanzerösen oder präkanzerösen Krankheit, die mit einer Hinaufregulation der PDGFR-beta-Spiegel einhergeht, umfassend:
(a) den immunologischen Nachweis und die Quantifizierung des durchschnittlichen Spiegels an Gesamt-PDGFR-β-Protein in Serum- oder Plasmaproben, die von Individuen einer Kontrollpopulation entnommen wurden, mit Antikörpern, die an Gesamt-PDGFR-β-Protein binden;
(b) den immunologischen Nachweis und die Quantifizierung von seriellen Veränderungen in den Gesamt-PDGFR-β-Protein-Spiegeln in Serum- oder Plasmaproben, die von dem Patienten entnommen wurden, über die Zeit mit Antikörpern, die an Gesamt-PDGFR-β-Protein binden;
(c) Vergleichen der Spiegel an Gesamt-PDGFR-ß-Protein in den Patientenproben mit dem durchschnittlichen Spiegel an Gesamt-PDGFR-β-Protein in den Kontrollproben und
(d) Entscheiden, ob eine herkömmliche Therapie und/oder eine auf einen PDGF-Weg abzielende Therapie zur Behandlung des Patienten verwendet wird, anhand der Unterschiede zwischen den Spiegeln an Gesamt-PDGFR-ß-Protein in den Patientenproben und dem durchschnittlichen Spiegel an Gesamt-PDGFR-ß-Protein in den Kontrollproben und im Hinblick auf die seriellen Veränderungen zwischen den einzelnen Spiegeln an Gesamt-PDGFR-ß-Protein in den Patientenproben,
wobei Spiegel, die über den durchschnittlichen Spiegel an Gesamt-PDGFR-ß-Protein in den Kontrollproben hinaus tendieren, auf eine durch PDGF angetriebene Krankheit hinweisen und die Entscheidung gefällt werden kann, eine auf einen PDGF-Weg abzielende Therapie zur Behandlung des Patienten zu verwenden.

4. Verfahren nach den Ansprüchen 1, 2 und 3, wobei die Therapie aus Multi-Kinaseinhibitoren, Tyrosinkinase-Inhibitoren, monoklonalen Antikörpern und Bisarylharnstoffen ausgewählt wird.

5. Verfahren nach den Ansprüchen 1, 2 und 3, wobei die Therapie eine auf einen PDGF-Weg abzielende Therapie ist.

6. Verfahren nach Anspruch 5, wobei es sich bei der auf einen PDGF-Weg abzielenden Therapie um den Tyrosinkinase-Inhibitor Imatinib-Mesylat oder den Bisarylharnstoff Sorafenib (BAY 43-9006) handelt.

7. Diagnoseverfahren zum Nachweisen einer kanzerösen oder präkanzerösen Krankheit, die mit einer Hinaufregulation der PDGFR-beta-Spiegel einhergeht, in einem menschlichen Individuum, umfassend:
(a) den immunologischen Nachweis und die Quantifizierung des durchschnittlichen Spiegels an Gesamt-PDGFR-ß-Protein in Serum- oder Plasmaproben, die von Individuen einer Kontrollpopulation entnommen wurden, mit Antikörpern, die an Gesamt-PDGFR-ß-Protein binden;
(b) den immunologischen Nachweis und die Quantifizierung von seriellen Veränderungen in den Gesamt-PDGFR-ß-Protein-Spiegeln in Serum- oder Plasmaproben, die von dem Individuum entnommen wurden, über die Zeit mit Antikörpern, die an Gesamt-PDGFR-ß-Protein binden; und
(c) Vergleichen der Spiegel an Gesamt-PDGFR-ß-Protein in den Proben des Individuums mit dem durchschnittlichen Spiegel an Gesamt-PDGFR-ß-Protein in den Kontrollproben, wobei ein Spiegel an Gesamt-PDGFR-ß-Protein in den Proben des Individuums, der über dem durchschnittlichen Spiegel an Gesamt-PDGFR-ß-Protein in den Kontrollproben liegt, auf einen aktivierten PDGF-Weg und das Vorliegen einer Krankheit in dem Individuum hinweist.

8. Verfahren nach den Ansprüchen 3 und 7, wobei der immunologische Nachweis und die Quantifizierung in den Schritten (a) und (b) mittels Sandwich-Immunassay erfolgen.

9. Verfahren nach den Ansprüchen 2 und 8, wobei der Sandwich-Immunassay die Verwendung von einem oder mehreren monoklonalen Antikörper(n) umfasst, der/die selektiv an die Ektodomäne des PDGFR-ß-Proteins binden.

10. Verfahren nach Anspruch 1, wobei die präneoplastische/neoplastische Krankheit aus der Gruppe ausgewählt ist, die aus metastatischem Medulloblastom, Dermatofibrosarcoma protuberans, gastrointestinalen Stroma-Tumoren, Kolorektalkrebs, Kolonkrebs, Lungenkrebs, nicht-kleinzelligem Lungenkrebs, kleinzelligem Lungenkrebs, chronischen myeloproliferativen Krankheiten, akuter myeloischer Leukämie, Schilddrüsenkrebs, Bauchspeicheldrüsenkrebs, Harnblasenkrebs, Nierenkrebs, Melanom, Brustkrebs, Prostatakrebs, Ovarialkrebs, Gebärmutterhalskrebs, Kopf- und Halskrebs, Gehirntumoren, hepatozellulärem Karzinom, hämatologischen Malignitäten und Prä-Krebserkrankungen, die zu den vorstehend genannten Krebserkrankungen führen, besteht.

11. Verfahren nach den Ansprüchen 1, 3 und 7, die außerdem prognostisch für die Krankheit sind, wobei die Spiegel an Gesamt-PDGFR-ß-Protein in den Patientenproben auf eine bessere oder schlechtere Prognose für den Patienten hinweisen.

12. Verfahren nach Anspruch 11, wobei die Prognose ein klinisches Resultat ist, das aus der Gruppe ausgewählt ist, die aus Ansprechrate (response rate, RR), vollständigem Ansprechen (complete response, CR), partiellem Ansprechen (partial response, PR), stabiler Krankheit (stable disease, SD), Zeit bis zur Krankheitsprogression (time to progression, TTP), progressionsfreier Überlebenszeit (progression-free survival, PFS), gesamter Überlebenszeit (overall survival, OS) und klinischem Nutzen, der vollständiges Ansprechen (CR), partielles Ansprechen (PR) und stabile Krankheit (SD) umfasst, besteht.

13. Verfahren nach Anspruch 11, wobei ansteigende Spiegel an Gesamt-PDGFR-ß auf eine größere Möglichkeit einer frühen Rekurrenz von Metastasen hindeuten.

14. Verfahren nach den Ansprüchen 1, 3 und 7, wobei es sich bei den Patientenproben um Proben vor der Behandlung handelt.

15. Verfahren nach den Ansprüchen 1, 3 und 7, das außerdem die Verwendung eines Immunassays zum Nachweisen oder Nachweisen und Quantifizieren der Spiegel von einem oder mehreren anderen Proteinen in den Proben des Individuums umfasst.

16. Verfahren nach Anspruch 15, wobei das andere Protein oder die anderen Proteine aus der Gruppe ausgewählt ist/sind, die aus Inhibitoren, Onkoproteinen, Wachstumsfaktor-Rezeptoren, angiogenen Faktoren, Metastaseproteinen, Tumormarkern und Tumorsuppressoren besteht.

17. Verfahren nach Anspruch 16, wobei es sich bei dem Inhibitor um den Gewebe-Inhibitor von Metalloproteinase-1 (TIMP-1) handelt, die Onkoproteine aus der Gruppe ausgewählt sind, die aus Her-2/neu und ras p21 besteht, die Wachstumsfaktor-Rezeptoren aus der Gruppe ausgewählt sind, die aus Epidermis-Wachstumsfaktor-Rezeptor (EGFR) und aus Plättchen stammendem Wachstumsfaktor-Rezeptor α (PDGFR-α) besteht, der angiogene Faktor Gefäßendothel-Wachstumsfaktor (VEGF) ist, das Metastaseprotein Urokinasetyp-Plasminogenaktivator (uPA) ist, der Tumormarker karzino-embryonales Antigen (CEA) ist und der Tumorsuppressor p53 ist.

18. Verfahren nach Anspruch 15, wobei es sich bei dem anderen Protein um PDGFR-α-Protein handelt.

19. Verfahren nach den Ansprüchen 1 und 3, wobei die Körperflüssigkeitsproben von einem Krebspatienten stammen, der nicht auf die Behandlung angesprochen hat.

## Revendications

1. Procédé de contrôle de l'état d'une maladie cancéreuse ou précancéreuse associée à une surégulation des taux de PDGFR-béta chez un patient et/ou de contrôle de la manière dont un patient ayant cette maladie réagit à un traitement, dans lequel on détecte immunologiquement et on quantifie des variations en série des taux totaux de protéine PDGFR-β dans des échantillons de sérum ou de plasma prélevés du patient en fonction du temps sur des anticorps fixant la protéine des PDGFR-β totale ;
dans lequel des taux croissants de protéine de PDGFR-β totale en fonction du temps indique une progression de la maladie ou une réaction négative au traitement, et dans lequel des taux décroissants de protéine de PDGFR-β totale en fonction du temps indique une rémission de la maladie ou une réaction positive au traitement.

2. Procédé suivant la revendication 1, dans lequel la détection immunologique et la quantification s'effectuent par un immunodosage sandwich.

3. Procédé de sélection d'un traitement d'un patient humain ayant une maladie cancéreuse ou précancéreuse associée à une surégulation des taux de PDGFR-β, comprenant :
(a) on détecte immunologiquement et on quantifie le taux moyen de protéine PDGFR-β totale dans des échantillons de sérum ou de plasma pris chez des individus d'une population témoin sur des anticorps fixant la protéine de PDGFR-β totale ;
(b) on détecte immunologiquement et on quantifie des variations en série de taux de protéine de PDGFR-β totale dans des échantillons de sérum ou de plasma pris chez le patient en fonction du temps sur des anticorps fixant la protéine de PDGFR-β totale ;
(c) on compare les taux de protéine de PDGFR-β totale dans les échantillons du patient au taux moyen de protéine PDGFR-β totale dans les échantillons témoins ; et
(d) on décide si l'on utilise un traitement classique et/ou un traitement visant la voie PDGF pour traiter le patient sur la base des différences entre les taux de protéine de PDGFR-β totale dans les échantillons du patient et le taux moyen de protéine de PDGFR-β totale dans les échantillons témoins et aux vues des variations en série parmi les taux de protéine de PDGFR-β totale dans les échantillons du patient,
dans lequel des taux qui tendent à être supérieurs au taux moyen de protéine de PDGFR-β totale dans les échantillons témoins indiquent une maladie commandée par PDGF, et on peut prendre la décision d'utiliser un traitement visant la voie PGDF pour traiter le patient.

4. Procédé suivant les revendications 1, 2 et 3, dans lequel on choisit le traitement parmi des inhibiteurs de multikinase, des inhibiteurs de tyrosine kinase, des anticorps monoclonaux et des bis-aryle urée.

5. Procédé suivant les revendications 1, 2 et 3, dans lequel le traitement est un traitement visant la voie PGDF.

6. Procédé suivant la revendication 5, dans lequel le traitement visant la voie PGDF est l'inhibiteur de tyrosine kinase mesylate d'imatinibe ou la bis-aryle urée Sorafenibe ( BAY 43-9006 ).

7. Procédé de diagnostic pour détecter une maladie cancéreuse ou précancéreuse associée à une surégulation de taux de PDGFR-β chez un sujet humain, dans lequel :
( a ) on détecte immunologiquement et on quantifie le taux moyen de protéine PDGFR-β totale dans des échantillons de sérum ou de plasma pris chez des individus d'une population témoin sur des anticorps fixant la protéine de PDGFR-5 totale ;
( b ) on détecte immunologiquement et on quantifie des variations en série de taux de protéine de PDGFR-β totale dans des échantillons de sérum ou de plasma pris chez le sujet en fonction du temps sur des anticorps fixant la protéine de PDGFR-β totale ; et
( c ) on compare les taux de protéine de PDGFR-β totale dans les échantillons du sujet au taux moyen de protéine de PDGFR-β totale dans les échantillons témoins ; un taux de protéine de PDGFR-β totale dans les échantillons du sujet qui est supérieur au taux moyen de protéine de PDGFR-β totale dans les échantillons témoins indiquant une voie activée de PDGFR et la présence de la maladie chez le sujet.

8. Procédé suivant les revendications 3 et 7, dans lequel on effectue la détection immunologique et la quantification des stades ( a ) et ( b ) par un immunodosage sandwich.

9. Procédé suivant les revendications 2 et 8, dans lequel l'immunodosage sandwich comprend l'utilisation d'un ou de plusieurs anticorps monoclonaux qui fixent sélectivement l'ectodomaine de la protéine PDGFR-β totale.

10. Procédé suivant la revendication 1, dans lequel la maladie prénéoplastique/néoplastique est choisie dans le groupe consistant en le médulloblastome métastatique, en le dermatofibrome de Darier-Ferrand, en des tumeurs stromales gastrointestinales, en un cancer colorectal, en un cancer du colon, en un cancer du poumon, en un cancer du poumon de cellules non petites, en un cancer du poumon de cellules petites, en des maladies myeloprolifératives chroniques, en des leucémies myelogènes aigus, en un cancer de la tyroïde, en un cancer du pancréas, en un cancer de la vésicule, en un cancer du rein, en un mélanome, en un cancer du sein, en un cancer de la prostate, en un cancer des ovaires, en un cancer de la tête et du cou, en des tumeurs du cerveau, en un carcinome hépatocellulaire, en des tumeurs malignes hématologiques et en des précancers conduisant aux cancers mentionnés ci-dessus.

11. Procédé suivant les revendications 1, 3 et 7 qui sont en outre un pronostic de la maladie, les taux de protéine de PDGFR-β totale dans les échantillons du patient indiquant un pronostic meilleur ou moins bon pour le patient.

12. Procédé suivant la revendication 11, dans lequel le pronostic est un résultat clinique choisi dans le groupe consistant en une vitesse ( RR ) de réaction, en une réaction ( CR ) complète, en une réaction ( PR ) partielle, en une maladie ( SD ) stable, en une durée d'évolution ( TTP ), en une survie sans évolution ( PFS ), en une survie globale ( OS ), et en bénéfice clinique, qui comprend une réaction ( CR ) complète, une réaction ( PR ) partielle, et une maladie ( SD ) stable.

13. Procédé suivant la revendication 11, dans lequel des taux croissants de PDGFR-β totale indiquent une probabilité plus grande de rechute précoce ou de métastase.

14. Procédé suivant les revendications 1, 3 et 7, dans lequel les échantillons du patient sont des échantillons de prétraitement.

15. Procédé suivant les revendications 1, 3 et 7, comprenant en outre l'utilisation d'un immunodosage pour détecter ou pour détecter et quantifier des taux d'une autre protéine ou de plusieurs autres protéines dans les échantillons du sujet.

16. Procédé suivant la revendication 15, dans lequel l'autre protéine ou les autres protéines est ou sont choisis dans le groupe consistant en des inhibiteurs, des oncoprotéines, des récepteurs de facteurs de croissance, des facteurs angiogène, des protéines métastasiques, des marqueurs de tumeurs, et des supresseurs de tumeurs.

17. Procédé suivant la revendication 16, dans lequel l'inhibiteur est inhibiteur de tissus de métalloprotéinase-1 ( TIMP-1 ), les oncoprotéines sont choisies dans le groupe consistant en HER-2/nouveau et ras p21, les récepteurs de facteurs de croissance sont choisis dans le groupe consistant en un récepteur épidermique de facteur de croissance ( EGFR ) et en un alpha récepteur de facteur de croissance dérivé des plaquettes ( PDGFR-α ), le facteur angiogène est un facteur de croissance endothéliale vasculaire ( VEGF ), la protéine métastasique est un activateur de plasminogène de type urokinase ( uPA ), le marqueur de tumeur est un antigène carcinoembryonique ( CEA ), et le supresseur de tumeur est p53.

18. Procédé suivant la revendication 15, dans lequel l'autre protéine est une protéine de PDGFR-α.

19. Procédé suivant la revendication 1 et 3, dans lequel les échantillons de fluide corporel proviennent d'un patient cancéreux qui n'a pas réagi au traitement.
